# EUROPÄISCHE PATENTSCHRIFT

(11) **EP 0 619 097 B1**
(45) Veröffentlichungstag und Bekanntmachung des Hinweises auf die Patenterteilung: **23.06.1999**
(21) Anmeldenummer: 93810252.2
(22) Anmeldetag: 07.04.1993
(51) Int. Cl.: A61B 17/16, A61F 2/46

(54) **Raspel und ein damit kuppelbares Griffteil zum Vorbereiten eines Röhrenknochens für den Einsatz einer Prothese**
Rasp and detachable handle for preparing the medullary canal of a bone which is to receive a prosthesis
Râpe et manche accouplable pour la préparation du canal médullaire d'un os destiné à recevoir une prothèse

(43) Veröffentlichungstag der Anmeldung: 12.10.1994
(73) Patentinhaber: Sulzer Orthopädie AG, 6340 Baar (CH)
(72) Erfinder: Hansjörg, Koller, CH-8400 Winterthur (CH); Tregoning Miller, Anne, Cambridge / CB2 2EZ (GB)
(74) Vertreter: Trieblnig, Adolf

(56) Entgegenhaltungen:
- EP-A- 0 122 670
- WO-A-89/04238
- FR-A- 426 528
- FR-A- 2 656 519
- FR-A- 2 659 042
- US-A- 4 508 005
- US-A- 5 002 581
- US-A- 5 089 003

## Beschreibung

Die Erfindung betrifft eine Vorrichtung zum Vorbereiten eines Röhrenknochens für den Einsatz eines mit einer künstlichen Gelenkkugel zu bestückenden Implantatschaftes, insbesondere eines Femurschafts einer Hüftgelenksprothese, entsprechend dem Oberbegriff des Patentanspruchs 1.

Eine aus der EP-A-0 122 670 bekannte Vorrichtung der genannten Art enthält eine Raspel mit einem dem Implantatschaft entsprechenden Schaftkörper und einer von diesem abstehenden zylindrischen Halspartie und einen mit der Raspel kuppelbaren Griffteil, der eine an die Halspartie ansetzbare Halterung und eine mit der Halspartie lösbar verbindbare Verriegelungseinrichtung aufweist. Die Halterung enthält zwei an die Halspartie seitlich anlegbare Greifbacken, welche je mit einer halbzylindrischen Vertiefung zur Aufnahme eines entsprechenden Teils der Halspartie versehen sind. Die eine der Greifbacken ist mit dem Griffteil fest verbunden und an ihrer Innenseite mit einer in eine Ringnut der Halspartie einführbaren halbringförmigen Rippe versehen, über welche jeweils die Raspel mit der angesetzten Halterung verriegelt werden soll. Die zweite Greifbacke ist an der festen Greifbacke über eine schwalbenschwanzförmige Führung in axialer Richtung der Halspartie beweglich zwischen einer die Halspartie im Eingriff haltenden Sperrstellung und einer die Halspartie freigebenden Lösestellung verstellbar geführt. Zur Begrenzung der Verschiebebewegungen ist an der festen Greifbacke ein Stift befestigt, der in einen in der beweglichen Greifbacke ausgebildeten Schlitz eingreift. Die Greifbacken werden jeweils auf den Schaftkörper aufgesetzt, mit der Halspartie zusammengeführt und in einer Sperrstellung verriegelt, in der zwei an den Greifbacken ausgebildete Vorsprünge je in eine am Schaftkörper ausgebildete Nut eingreifen, um die Kupplungsverbindung zu sichern. Derartige, mit mehreren beweglichen Teilen ausgeführte Vorrichtungen erfordern einen relativ grossen Arbeits- und Zeitaufwand bei der Herstellung und beim Zusammenbau sowie beim Reinigen und Sterilisieren der Vorrichtung.

Der Erfindung liegt die Aufgabe zugrunde, eine insbesondere in dieser Hinsicht weiter entwickelte Vorrichtung der eingangs genannten Gattung in einer gegenüber bisherigen Ausführungen vereinfachten, kompakten Bauweise zu schaffen, welche wenige, robust ausführbare und leicht zu handhabende Bauteile enthält und welche mit entsprechend geringem Arbeits- und Zeitaufwand die Vorbereitung und Reinhaltung des Implantationsbereichs gewährleistet.

Diese Aufgabe wird durch die im kennzeichnenden Teil des Patentanspruchs 1 angegebenen Merkmale gelöst.

Ein wesentlicher Vorteil der erfindungsgemässen Ausführung besteht darin, dass die zum Einschlagen der Raspel über den Griffteil einzuleitende Anpresskraft an der Stirnseite der Halspartie, und damit an der vom Knochengewebe am weitesten entfernten Stelle auf die Raspel übertragen wird, so dass der Griffteil vom Schaftkörper und vom Knochengewebe ferngehalten und ständig eine ungehinderte Beobachtung des Implantationsbereichs gewährleistet werden kann. Ein weiterer Vorteil der erfindungsgemässen Ausführung besteht darin, dass die Führungshülse mit dem dem Schaftkörper zugewandten Endabschnitt des Griffteils zu einem handlichen, kompakten Werkzeug verbunden sein kann. Der am Griffteil schwenkbar angelenkte Kupplungsteil ist leicht zugänglich und ermöglicht die Verwendung einer einfach ausgebildeten und einfach betätigbaren Verstellanordnung zum Kuppeln und Entkuppeln des Kupplungsteils mit der bzw. von der Halspartie. Die Erfindungsgemässe Vorrichtung zeichnet sich insbesondere aus durch einfach herstellbare und einfach zusammenbaubare Teile, die leicht gereinigt und sterilisiert werden können.

In den abhängigen Patentansprüchen sind Ausgestaltungen des Erfindungsgegenstandes angegeben.

Die Ausführung nach Anspruch 2 ermöglicht auf einfache Weise und mit geringem Zeitaufwand eine genaue Vorbestimmung der Position der zu implantierenden Gelenkkugel unter Verwendung von Probierkugeln mit unterschiedlich tief in das Kugelinnere sich erstreckenden Konusflächen, so dass mit einem Satz von Probierkugeln mit z.B. millimeterweise abgestuften Eindringtiefen auch bei vollständig eingeschlagenem Schaftkörper die Probierkugel in einem durch die anatomischen Verhältnisse gegebenen, passenden Abstand vom Schaftkörper positioniert und probeweise mit der zugeordneten Gelenkschale zusammengeführt werden kann. Durch die im Anspruch 2 weiter hervorgehobene Positionierung der zylindrischen und konischen Führungsflächen der Halspartie bezüglich der an dieser ausgebildeten Stützfläche sowie der Führungsflächen der Bohrung der Führungshülse bezüglich der am Griffteil ausgebildeten Anschlagfläche ist einerseits eine genaue Führung der mit geringem Spiel auf die Halspartie 3 aufsetzbaren Führungshülse erzielbar, andererseits wird ein Festsetzen der Führungshülse auf der Halspartie verhindert und damit eine Beschädigung insbesondere der für die genaue Positionierung der Probierkugeln wesentlichen Konusfläche der Halspartie vermieden.

Weitere Einzelheiten und Merkmale ergeben sich aus der folgenden Beschreibung eines in der Zeichnung schematisch dargestellten Ausführungsbeispiels nach der Erfindung, in Verbindung mit den Patentansprüchen. Es zeigen:
- Fig. 1: Eine erfindungsgemäss ausgebildete Vorrichtung zum Vorbereiten eines Oberschenkelknochens, teilweise in einem Längsschnitt dargestellt;
- Fig. 2: eine Einzelheit der Vorrichtung nach Fig. 1 in einer entsprechenden Teilansicht mit Teilschnitt und in einer grösseren Darstellung und
- Fig. 3: eine Seitenansicht der Einzelheit mit einem Teilschnitt entsprechend der Linie III-III in Fig. 2;
- Fig. 4: eine weitere erfindungsgemäss ausgebildete Vorrichtung nach einer abgewandelten Ausführungsform;
- Fig. 5: eine Einzelheit der Vorrichtung nach Fig. 4 in einer grösseren Darstellung, und
- Fig. 6: eine Seitenansicht der Einzelheit mit einem Teilschnitt entsprechend der Linie VI-VI in Fig. 5.

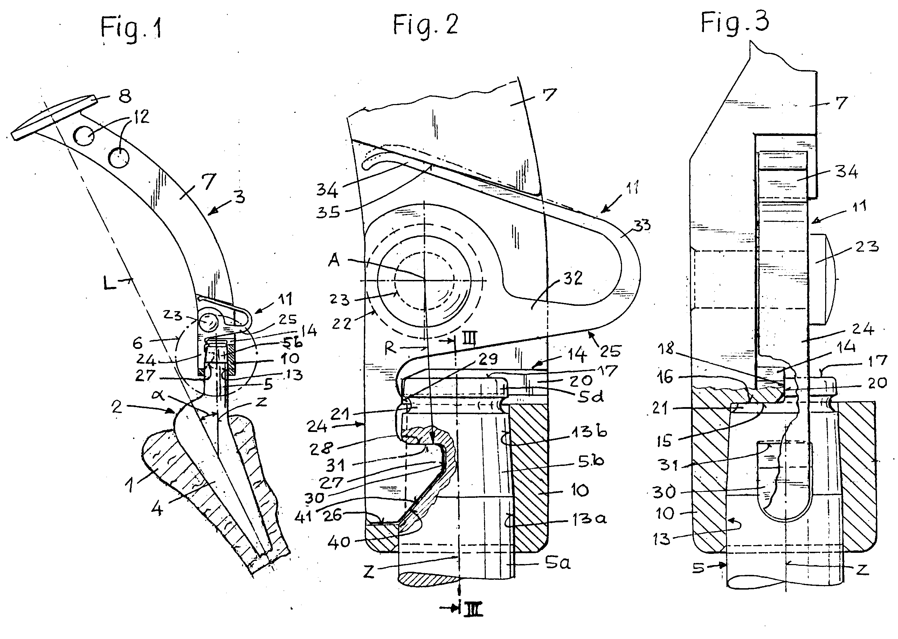

Die Vorrichtung nach Fig. 1 enthält ein zum Einschlagen in einen Röhrenknochen bestimmtes, darstellungsgemäss in einen Oberschenkelknochen 1 eingeschlagenes Räuminstrument in Form einer Raspel 2 und ein mit dieser kraft- und formschlüssig kuppelbares Hilfswerkzeug 3 zum Führen der Raspel 2 beim Einschlagen in das Knochengewebe und beim Zurückziehen aus dem Knochengewebe. Die Raspel 2 ist mit einem bezüglich seiner Längsachse L konisch sich verjüngenden, in das Knochengewebe einschlagbarem Schaftkörper 4 und einer von diesem abstehenden Halspartie 5 ausgeführt, die einen zylindrischen Führungsabschnitt 5a und einen bezüglich einer Zentrierachse Z sich verjüngenden konischen Führungsabschnitt (Konusfläche) 5b aufweist. Die Zentrierachse Z ist darstellungsgemäss unter einem Winkel α zur Längsachse L des Schaftkörpers 4 geneigt. Der Schaftkörper 4 ist in bekannter Weise mit nicht dargestellten, über seine Arbeitslänge, auf dem Umfang verteilten Zähnen versehen, die beim Einschlagen in Richtung der Längsachse L Knochenmaterial abschälen.

Der Schaftkörper 4 entspricht in Form und Grösse einem mit einer Gelenkkugel zu bestückenden, nicht dargestellten Implantatschaft einer Hüftgelenksprothese. Die Halspartie 5 dient als Aufsteckdorn für das Hilfswerkzeug 3 sowie für eine der Gelenkkugel entsprechende, in Fig. 1 strichpunktiert dargestellte Probierkugel 6, die mit einer auf den Führungsabschnitt 5b passenden konischen Aufnahmebohrung ausgeführt ist. Zur genauen Bestimmung der den jeweiligen anatomischen Verhältnissen entsprechenden Einbaulage der zu implantierenden Gelenkkugel kann in bekannter Weise aus einem Satz von mit unterschiedlich tiefen, z.B. millimeterweise abgestuften Aufnahmebohrungen ausgeführten Probierkugeln 6 eine der Probierkugeln ausgewählt, auf den Führungsabschnitt 5b selbsthemmend aufgesetzt und probeweise mit der nicht dargestellten Hüftgelenkspfanne zusammengeführt werden. Entsprechend kann durch Aufsetzen von Probierkugeln 6 mit verschieden tiefen Aufnahmebohrungen die endgültige Einbaulage der zu implantierenden Gelenkkugel und damit der Drehpunkt am Femur genau vorbestimmt werden.

Das Hilfswerkzeug 3 enthält einen Griffteil 7, der an einem Ende mit einem Kopfstück 8 und am anderen Ende mit einer auf den Endabschnitt 5a aufsteckbaren Führungshülse 10 und einem mit der Halspartie 5 zusammenführbaren Kupplungsteil 11 versehen ist. Der Griffteil 7 kann darstellungsgemäss als bogenförmiger Halter ausgebildet sein, der die auf die abgewinkelte Halspartie 5 aufsetzbare Führungshülse 10 mit dem beim dargestellten Beispiel in der Fortsetzung der Längsachse L positionierbaren Kopfstück 8 verbindet. Das Kopfstück 8 kann mit einer sphärischen Ambossfläche für ein Werkzeug zum Einschlagen des Schaftkörpers 4 versehen sein, dessen Anpresskraft im wesentlichen in Richtung der Längsachse L in den Schaftkörper eingeleitet wird. Am Griffteil 7 können ferner Bohrungen 12 zum Ansetzen eines Werkzeugs, z.B. eines Querstabes zum Zurückziehen des Schaftköpers 4 vorgesehen sein.

Wie insbesondere aus den Fig. 2 und 3 hervorgeht, ist die Führungshülse 10 mit einer die Halspartie 5 aufnehmenden Bohrung 13 ausgeführt, die einen am schaftseitigen Ende der Führungshülse ausgebildeten zylindrischen Abschnitt 13a als Führungsfläche für den Führungsabschnitt 5a und eine dem konischen Führungsabschnitt 5b der Halspartie 5 zugeordnete Führungsfläche in Form eines entsprechend konischen Abschnitts 13b aufweist. Nach einer anderen, nicht dargestellten Ausführungsform kann eine entsprechende Führungshülse auch mit einer über ihre ganze Länge konisch verlaufenden Bohrung 13 versehen sein. Der Griffteil 7 ist mit einer dem Ende der Halspartie 5 zugeordneten Aufsetzpartie 14 ausgeführt, welche mindestens einen Teil, darstellungsgemäss die Hälfte, des Querschnitts der Bohrung 13 überdeckt und welche mit einer quer zur Zentrierachse Z verlaufenden Anschlagfläche 15 auf eine entsprechende, an der Stirnseite der Halspartie 5 ausgebildete Stützfläche 16 aufsetzbar ist, so dass die Eindringtiefe der Halspartie 5 in der Führungshülse 10 begrenzt wird. Wie insbesondere aus der Fig. 3 hervorgeht, kann die Stützfläche 16 durch einen zurückgesetzten Abschnitt der Stirnfläche 17 der Halspartie 5 gebildet und durch eine Schulter begrenzt sein, die eine parallel zur Zentrierachse Z verlaufende Flanke 18 bildet. An einer Schulter der Aufsetzpartie 14 kann eine entsprechende, mit der Flanke 18 zusammenführbare Gegenfläche 20 ausgebildet sein, so dass der Griffteil 7 nur in einer definierten Winkelstellung auf die Halspartie 5 aufgesetzt werden kann.

Die Konusflächen 5b und 13b der Halspartie 5 und der Bohrung 13 sind in Richtung der Zentrierachse Z zueinander und je bezüglich der Stützfläche 16 der Halspartie 5 bzw. der Anschlagfläche 15 des Griffteils 7 so positioniert, dass bei auf die Stützfläche 16 aufgesetztem Griffteil 7 die zum Einschlagen des Schaftkörpers 4 auf diesen zu übertragende Anpresskraft im wesentlichen über die Stützfläche 16 eingeleitet wird und die Halspartie 5 in der Bohrung 13 mit einem Spiel geführt ist, welches eine Zentrierung der Führungshülse 10 auf der Halspartie 5 gewährleistet und welches zugleich auch nach Erreichen der beim Einschlagen des Schaftkörpers von der Anschlagfläche 15 auf die Stützfläche 16 zu übertragenden maximalen Anpresskraft ein Lösen der Konusflächen 5b und 13b ohne Selbsthemmung gestattet. Entsprechend wird eine Beschädigung der jeweils mit einer der Probierkugeln 6 zu bestückenden Konusfläche 5b der Halspartie 5 vermieden. Durch die beim Einschlagen der Raspel 2 auf die Halspartie 5 einwirkenden Schläge können in bestimmten Fällen Stauchpartien entstehen, die seitlich aus dem Bereich der Stützfläche 16 austreten. Um eine Beschädigung der relativ empfindlichen Konusflächen 5b und 13b durch derartige Stauchpartien zu vermeiden, kann darstellungsgemäss der Endabschnitt der Halspartie 5 im Bereich der Stützfläche 16 mit einer ringförmigen Entlastungsnut 21 ausgeführt sein, durch welche derartige Stauchpartien aufgenommen und von den Konusflächen 5b und 13b ferngehalten werden können.

Der Kupplungsteil 11 ist als einstückiger Bauteil, in Form einer Sperrklinke ausgeführt, die am Griffteil 7 um einen an diesem befestigten, z.B. eingepressten Stift 23 schwenkbar angelenkt ist. Die Sperrklinke ist nach Art eines zweiarmigen Winkelhebels mit einem in Richtung der Zentrierachse Z gegen das schaftseitige Ende der Führungshülse 10 orientierten Klinkenarm 24 und einem quer dazu gestellten Betätigungsarm 25 versehen. Der Klinkenarm 24 erstreckt sich gegen eine die Wand der Führungshülse 10 über einen Teil ihrer Länge durchsetzende Ausnehmung 26 und weist eine durch diese in die Bohrung 13 der Führungshülse 10 einführbare und aus dieser ausschwenkbare Klaue 27 auf. Die Klaue 27 ist mit einer der Anschlagfläche 15 des Griffteils 7 zugewandten Flanke 28 und einer der Anschlagfläche 15 abgewandten Flanke 40 ausgeführt. Die Flanke 28 ist darstellungsgemäss in Form einer zylindrischen Fläche ausgebildet, deren Krümmungsradius R dem Abstand der Flanke 28 von der Schwenkachse A des Kupplungsteils 11 entspricht. Die Flanke 40 ist in Form einer gegen das Ende der Führungshülse 10 geneigten, schrägen Auflauffläche ausgeführt, welche zum Zusammenwirken mit dem Ende der Halspartie 5 bestimmt ist. Beim Aufstecken der Führungshülse 10 auf die Halspartie 5 wird die unter Federwirkung gehaltene Klaue 27 - beim Zusammentreffen ihrer Auflauffläche mit der Stirnseite der Halspartie 5 - zunehmend aus einer Stellung, die etwa derjenigen nach Fig. 2 entspricht, im Uhrzeigersinn in eine Lösestellung verschwenkt, in der die Klaue 27 in axialer Richtung über den Führungsabschnitt 13b verschiebbar ist, bis sie in eine in der Halspartie 5 ausgebildete Ausnehmung 30 einrastet. Die Ausnehmung 30 weist eine der Flanke 28 entsprechend gekrümmte, dem Schaftkörper 4 zugewandte, hinterschnittene zylindrische Gegenflanke 31 und eine der Flanke 40 entsprechend geneigte Gegenflanke 41 auf. Die eingerastete Klaue 27 ist durch die zusammenwirkenden Flanken 28 und 31 gegen Lösen gesichert und kann erst durch manuelle Betätigung gelöst werden. Am Klinkenarm 24 kann darstellungsgemäss ein an die Aufsetzpartie 14 anlegbarer Anschlag 29 vorgesehen sein, der den Schwenkbereich des Klinkenarms 24 gegen die Halspartie 5, und damit die Eindringtiefe der Klaue 27 in der Ausnehmung 30 auf ein vorbestimmtes Mass begrenzt, so dass eine im wesentlichen anschlagfreie, die zusammenwirkenden Flächen schonende Einführung der Klaue 27 in die innerhalb der Ausnehmung 31 vorgesehene Sperrstellung erzielbar ist.

Der Betätigungsarm 25 weist einen annähernd rechtwinkelig vom Klinkenarm 24 abgewinkelten Anschlussabschnitt 32 auf, der über einen gebogenen, beim dargestellten Beispiel um einen Winkel von ca. 160° abgewinkelten mittleren Abschnitt 33 in einen konisch sich verjüngenden, als Federelement ausgebildeten Endabschnitt 34 übergeht, welcher unter Vorspannung an einen am Griffteil 7 ausgebildeten Anschlag 35 anlegbar ist und über welchen der Klinkelhebel 24 gegen die Aufsetzpartie 14 verspannbar ist. Entsprechend wird bei auf die Stützfläche 16 aufgesetztem und durch die Führungsfläche 18 zentriertem Griffteil 7 die Klaue 27 unter Federwirkung in die Sperrstellung innerhalb der Ausnehmung 30 gedrängt und festgehalten. Nach einer anderen, nicht dargestellten Ausführungsform kann anstelle des dargestellten, mit dem Klinkenarm 24 einstückig verbundenen Federelementes ein entsprechend wirkendes, zwischen dem Klinkenarm 24 und dem Griffteil 7 anbringbares separates Federelement vorgesehen sein.

Durch den in die dargestellte Sperrstellung einrastenden Klinkenarm 24 werden die Raspel 2 und der Griffteil 7 jeweils in der für das Einschlagen und das Zurückziehen der Raspel 2 bestimmten gegenseitigen Stellung fixiert. Zum Entkuppeln des Griffteils 7 von der Halspartie 5 kann der Betätigungsarm 25 der Sperrklinke durch eine entsprechende Fingerbewegung des den Griffteil haltenden Chirurgen in Fig. 2 im Uhrzeigersinn verschwenkt werden, so dass die Klaue 27 aus der dargestellten Sperrstellung in eine Lösestellung ausserhalb der Ausnehmung 30 geschwenkt wird, die ein Abheben des Griffteils 7 von der Halspartie 5 gestattet.

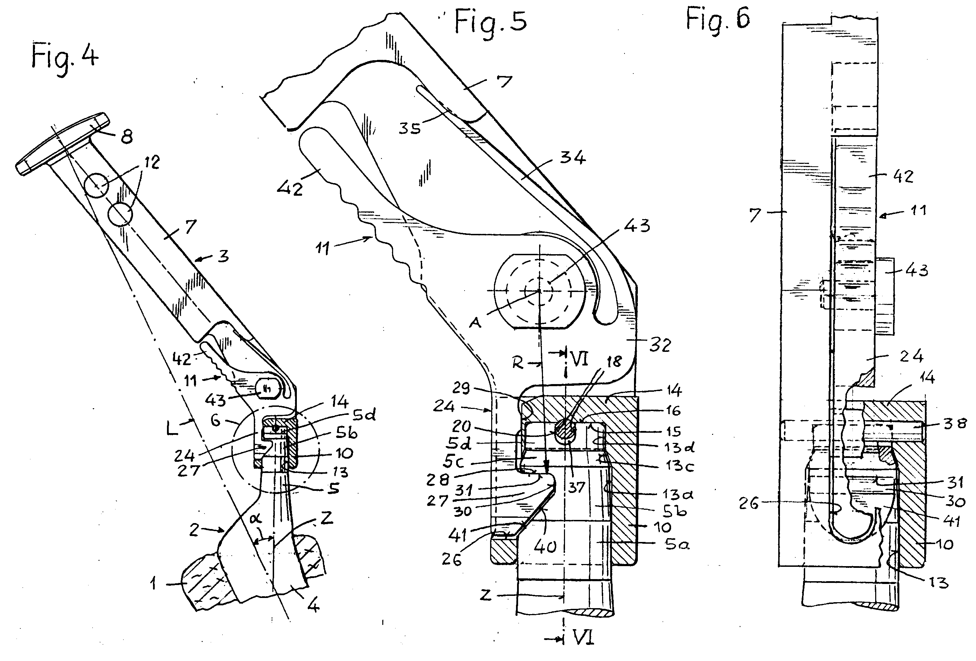

Bei der Vorrichtung nach den Fig. 4, 5 und 6 sind die der Ausführung nach den Fig. 1, 2 und 3 entsprechenden Teile mit den gleichen Bezugszeichen versehen. Gemäss Fig. 4 ist der Griffteil 7 in Form eines geraden Halters ausgebildet, und die Führungshülse 10 ist in Form eines Topfes ausgeführt, dessen Boden durch die Aufsetzpartie 14 gebildet ist, die bei dieser Ausführung den ganzen Querschnitt der Bohrung 13 überdeckt. Wie insbesondere aus den Fig. 5 und 6 hervorgeht, ist die Halspartie 5 mit einem an den konischen Führungsabschnitt 5b anschliessenden zweiten konischen Führungsabschnitt 5c und einem zylindrischen Endabschnitt 5d ausgeführt, dessen Stirnfläche als Stützfläche 16 ausgebildet und mit einer diese diametral durchsetzenden nutenartigen Vertiefung 37 versehen ist. Bei dieser Ausführung erstreckt sich der am schaftseitigen Ende der Führungshülse 10 ausgebildete zylindrische Abschnitt 13a der Bohrung 13 auch über den Längenabschnitt der Führungshülse 10, der den zum selbsthemmenden Aufstecken der Probierkugel 6 bestimmten konischen Führungsabschnitt 5b der Halspartie 5 aufnimmt. Dieser zylindrische Abschnitt 13a ist durch einen konischen Abschnitt 13c begrenzt, welcher eine Führungsfläche für den zweiten konischen Führungsabschnitt 5c der Halspartie 5 bildet und welcher in einen zweiten zylindrischen Abschnitt 13d übergeht, der eine Führungsfläche für den Endabschnitt 5d der Halspartie 5 bildet.

Die Führungsabschnitte 5c und 5d der Halspartie 5 und die Abschnitte 13a, 13c und 13d der Bohrung 13 sind je bezüglich der Stützfläche 16 bzw. der Anschlagfläche 15 ebenfalls so positioniert, dass bei auf die Stützfläche 16 aufgesetzter Führungshülse 10 der zylindrische Abschnitt 13a der Bohrung 13 den konischen Führungsabschnitt 5b berührungsfrei umgibt. Durch die zylindrischen Führungsabschnitte 5a und 5d und Bohrungsabschnitte 13a und 13d wird eine präzise Führung der Führungshülse 10 gewährleistet, welche jeweils durch die zusammenwirkenden konischen Flächen 5c und 13c zentriert und entsprechend schonend auf die Halspartie 5 aufgesetzt werden kann. Im Bereich der Aufsetzpartie 14 der Führungshülse 10 ist ein den Bohrungsabschnitt 13d diametral durchsetzender, z.B. eingepresster, Stift 38 angeordnet, der einen über die Anschlagfläche 15 vorstehenden, zwischen zwei in Richtung der Zentrierachse Z verlaufende, dargestellungsgemäss zueinander parallele Flanken 18 der Vertiefung 37 einführbaren Führungsteil mit den Nutenflanken 18 zugeordneten Gegenflanken 20 bildet. Entsprechend kann auch bei dieser Ausführung der Griffteil 7 nur in einer einzigen definierten Winkelstellung auf die Halspartie 5 aufgesetzt und mit dieser über den Kupplungsteil 11 verriegelt werden.

Der Kupplungsteil 11 nach den Fig. 5 und 6 ist mit einem dritten Hebelarm 42 ausgeführt und am Griffteil 7 mittels einer Schraube 43 angelenkt. Der dritte Hebelarm 42 ist als leicht zu handhabender Betätigungshebel ausgebildet, über den die Klaue 27 durch eine einfache Greifbewegung des Chirurgen aus der dargestellten Sperrstellung in die Lösestellung bzw. aus dieser in die Sperrstellung zurückgeschwenkt werden kann. Die Vertiefung 30 kann durch eine der Breite der Klaue 27 angepasste, z.B. in die Halspartie 5 eingefräste Nut (Fig. 3) oder durch einen entsprechenden, die Halspartie in Querrichtung durchsetzenden Einschnitt (Fig. 6) gebildet sein.

Bei den beschriebenen Ausführungen liegt der Klinkenarm 24 in der Sperrstellung innerhalb der umhüllenden Mantelfläche der Führungshülse 10. Entsprechend wird eine kompakte Bauweise der Vorrichtung erzielt, die aus wenigen einfachen, leicht zu reinigenden Bauteilen besteht und die insbesondere eine wirksame Abschirmung der zusammenwirkenden Flächen gegen ein Eindringen von Flüssigkeit und Knochenpartieln ermöglicht.

Zusammenfassend lässt sich somit die Erfindung wie folgt beschreiben:
Die Vorrichtung enthält eine dem Implantatschaft entsprechende Raspel 2, welche einen in das Knochengewebe einschlagbaren Schaftkörper 4 und eine davon abstehende, mit einer der Gelenkkugel entsprechenden Probierkugel 6 bestückbare Halspartie 5 aufweist, und einen mit der Raspel 2 kuppelbaren Griffteil 7 zum Einschlagen und Zurückziehen des Schaftkörpers 4. Der Griffteil 7 enthält eine auf die Halspartie 5 aufsteckbare Führungshülse 10 und eine Aufsetzpartie 14 mit einer auf eine stirnseitige Stützfläche 16 der Halspartie 5 aufsetzbaren Anschlagfläche 15. Am Griffteil 7 ist ein Kupplungsteil 11 angelenkt, der eine durch eine Ausnehmung 26 der Führungshülse 10 in eine Ausnehmung 30 der Halspartie 5 einrückbare und aus dieser ausrückbare Klaue 27 aufweist. Bei dieser aus einfachen, leicht steril zu haltenden Bauteilen herstellbaren Vorrichtung liegen alle beim Einschlagen und Zurückziehen des Schaftkörpers 4 wirksamen Anschlag-, Führungs- und Spannflächen innerhalb der umhüllenden Mantelfläche der Halspartie 5 und sind dadurch vor dem Kontakt mit Flüssigkeit und Knochenpartikeln geschützt.

## Patentansprüche

1. Vorrichtung zum Vorbereiten eines Röhrenknochens für den Einsatz eines mit einer künstlichen Gelenkkugel zu bestückenden Implantatschafts, insbesondere eines Femurschafts einer Hüftgelenkprothese, mit einer dem Implantatschaft bezüglich Form und Grösse entsprechenden Raspel (2), welche einen in Richtung ihrer Längsachse (L) in den Knochen (1) einschlagbaren und zurückziehbaren Schaftkörper (4) und eine von diesem abstehende, mit einer der Gelenkkugel entsprechenden Probierkugel (6) bestückbare Halspartie (5) aufweist, und einem auf die Raspel (2) aufsetzbaren Griffteil (7), der eine Hülse (10) mit einer Bohrung (13) zur Aufnahme der Halspartie (5) und eine mit der Halspartie (5) lösbar verbindbare Verriegelungseinrichtung enthält, wobei die Halspartie (5) mit einer stirnseitigen Stützfläche (16) für eine am Griffteil (7) ausgebildete, zum Einschlagen der Raspel (2) auf diese Stützfläche (16) aufsetzbare Anschlagfläche (15) ausgeführt ist und die Verriegelungseinrichtung einen am Griffteil (7) verstellbar angebrachten, mit der Halspartie (5) in Eingriff bringbaren und von dieser weg bewegbaren Kupplungsteil (11) enthält, dadurch gekennzeichnet, dass die Hülse (10) als eine auf die Halspartie (5) der Raspel (2) aufsteckbare Führungshülse ausgebildet ist und der Kupplungsteil (11) in Form einer am Griffteil (7) schwenkbar angelenkten Sperrklinke ausgeführt ist, welche eine zwischen einer Sperrstellung innerhalb einer an der Halspartie (5) der Raspel (2) ausgebildeten Ausnehmung (30) und einer Lösestellung ausserhalb dieser Ausnehmung verstellbare Klaue (27) enthält.

2. Vorrichtung nach Anspruch 1, dadurch gekennzeichnet, dass die Halspartie (5) mit einem zum Zusammenwirken mit einer konischen Aufnahmebohrung der Probierkugel (6) bestimmten, bezüglich einer Zentrierachse (Z) sich verjüngenden konischen Führungsabschnitt (5b) ausgeführt ist, dass ferner an der Halspartie (5) ein in die Führungshülse (10) einführbarer zylindrischer Führungsabschnitt (5a) ausgeführt ist, dass die Bohrung (13) der Führungshülse (10) eine dem zylindrischen Führungsabschnitt (5a) entsprechende zylindrische Führungsfläche (13a) enthält, und dass bei auf die Stützfläche (16) des Halsteils (5) aufgesetztem Griffteil die Halspartie (5) in der Bohrung (13) der Führungshülse (10) mit einem Spiel geführt ist, welches eine Zentrierung der Führungshülse (10) auf der Halspartie (5) gewährleistet und zugleich ein Lösen des konischen Führungsabschnitts (5b) ohne Selbsthemmung gestattet.

3. Vorrichtung nach Anspruch 2, dadurch gekennzeichnet, dass die Bohrung (13) der Führungshülse (10) mit einer dem konischen Führungsabschnitt (5b) der Halspartie (5) entsprechenden konischen Führungsfläche (13b) ausgeführt ist.

4. Vorrichtung nach Anspruch 2, dadurch gekennzeichnet, dass sich die zylindrische Führungsfläche (13a) der Bohrung (13) über einen den konischen Führungsabschnitt (5b) der Halspartie (5) aufnehmenden Längenabschnitt der Führungshülse (10) erstreckt.

5. Vorrichtung nach Anspruch 4, dadurch gekennzeichnet, dass die Halspartie (5) mit einem zweiten konischen Führungsabschnitt (5c) ausgeführt ist und dass die Bohrung (13) der Führungshülse (10) mit einer entsprechenden konischen Führungsfläche (13c) ausgeführt ist.

6. Vorrichtung nach einem der Ansprüche 2 bis 5, dadurch gekennzeichnet, dass die Halspartie (5) mit einem zylindrischen Endabschnitt (5d) ausgeführt ist, und dass die Bohrung (13) mit einer entsprechenden zweiten zylindrischen Führungsfläche (13d) ausgeführt ist.

7. Vorrichtung nach einem der vorangehenden Ansprüche, dadurch gekennzeichnet, dass die Anschlagfläche (15) des Griffteils (7) an einer mindestens einen Teil des Querschnitts der Bohrung (13) der Führungshülse (10) überdeckenden Aufsetzpartie (14) ausgebildet ist, und dass die Halspartie (5) mit mindestens einer quer zu ihrer Stützfläche (16) gestellten, in Richtung der Zentrierachse (Z) der Halspartie (5) verlaufenden Führungsfläche (18) für eine an der Aufsetzpartie (14) ausgebildete Gegenfläche (20) aufweist.

8. Vorrichtung nach Anspruch 7, dadurch gekennzeichnet, dass die Stützfläche (16) der Halspartie (5) an einem zurückgesetzten Abschnitt der Stirnfläche (17) ausgebildet ist, dass die Führungsfläche (18) an einer die Stützfläche (16) begrenzenden Schulter der Halspartie (5) ausgeführt ist, und dass die Gegenfläche (20) an einer entsprechenden Schulter der Aufsetzpartie (14) ausgeführt ist.

9. Vorrichtung nach Anspruch 7 oder 8, dadurch gekennzeichnet, dass die Führungsfläche (18) an einer Flanke einer in der Stützfläche (16) der Halspartie (5) ausgebildeten nutenartigen Vertiefung (37) ausgebildet ist, und dass die Gegenfläche (20) an einem von der Aufsetzpartie (14) abstehenden, in diese Vertiefung (37) einführbaren Führungsteil ausgebildet ist.

10. Vorrichtung nach einem der vorangehenden Ansprüche, dadurch gekennzeichnet, dass die Sperrklinke mit einem unter Vorspannung an einen am Griffteil (7) ausgebildeten Anschlag (35) anlegbaren Federelement (34) versehen und über dieses in der Sperrstellung verspannbar ist.

11. Vorrichtung nach Anspruch 10, dadurch gekennzeichnet, dass die Sperrklinke und das Federelement (34) an einem einstückigen Bauteil ausgebildet sind.

12. Vorrichtung nach einem der vorangehenden Ansprüche, dadurch gekennzeichnet, dass die Sperrklinke im Bereich einer in der Führungshülse (10) ausgebildeten Ausnehmung (26) angeordnet ist.

13. Vorrichtung nach einem der vorangehenden Ansprüche, dadurch gekennzeichnet, dass die Klaue (27) der Sperrklinke mit einer der Anschlagfläche (15) des Griffteils (7) zugekehrten Flanke (28) ausgeführt ist, die zum Zusammenwirken mit einer entsprechenden, an der Ausnehmung (30) der Halspartie (5) der Raspel (2) ausgebildeten, dem Schaftkörper (4) zugewandten Gegenflanke (31) bestimmt ist.

14. Vorrichtung nach Anspruch 13, dadurch gekennzeichnet, dass die Flanke (28) der Klaue (27) und die Gegenflanke (31) der Ausnehmung (30) der Halspartie (5) je in Form einer hinterschnittenen zylindrischen Fläche ausgebildet sind, die mit einem Krümmungsradius (R) ausgeführt ist, welcher dem Abstand der Flanke (28) von der Schwenkachse (A) der Sperrklinke entspricht.

15. Vorrichtung nach einem der vorangehenden Ansprüche, dadurch gekennzeichnet, dass die Klaue (27) der Sperrklinke mit einer der Anschlagfläche (15) des Griffteils (7) abgewandten Flanke (40) in Form einer schrägen Auflauffläche ausgeführt ist, welche zum Zusammenwirken mit dem Ende der Halspartie (5) beim Aufsetzen der Führungshülse (10) bestimmt ist und über welche die Klaue (27) beim Aufstecken der Führungshülse (10) auf die Halspartie (5) zunehmend in die Lösestellung verschwenkbar ist, die beim Zusammentreffen der Anschlagfläche (15) und der Stützfläche (16) ein Einrasten der Klaue (27) in die Ausnehmung (30) gestattet.

## Claims

1. A device for the preparation of a tubular bone for the insertion of an implant shaft to be equipped with an artificial ball-and-socket joint, in particular a femur shaft of a hip joint prosthesis, having a rasp (2) corresponding to the implant shaft with respect to shape and size, which comprises a shaft body (4) which can be driven into and withdrawn from the bone (1) in the direction of its longitudinal axis (L), and a neck part (5) protruding therefrom which can be provided with a test ball (6), corresponding to the ball-and-socket joint, and a handle (7) which can be placed on the rasp (2) and which contains a bush (10) having a bore (13) to contain the neck part (5) and a locking mechanism which can be detachably connected to the neck part (5), with the neck part (5) being designed with a front support face (16) for a stop face (15) which is constructed at the handle (7) and can be placed on said support face (16) to drive in the rasp (2) and the locking mechanism containing a coupling part (11) which is adjustably mounted on the handle (7), can be brought into engagement with the neck part (5) and can be moved away from it,
**characterised in that** the bush (10) is constructed as a guide bush which can be placed on the neck part (5) of the rasp (2) and the coupling part (11) is designed in the form of a detent pawl swivel-mounted on the handle (7), which contains a claw (27) adjustable between a locking position inside a recess (30) constructed on the neck part (5) of the rasp (2) and a disengaged position outside said recess.

2. A device according to Claim 1,
**characterised in that** the neck part (5) is designed with a conical guide portion (5b) intended to interact with a conical bore of test ball (6) and tapering in relation to a centring axis (Z),
**in that** a cylindrical guide portion (5a), which can be inserted into the guide bush (10), is also constructed on the neck part (5),
**in that** the bore (13) of guide bush (10) contains a cylindrical guide face (13a) corresponding to the cylindrical guide portion (5a),
**and in that** when the handle is placed on the support face (16) of the neck part (5), the neck part (5) is guided in the bore (13) of the guide bush (10) with a clearance which guarantees a centring of the guide bush (10) on the neck part (5) and at the same time permits a detachment of the conical guide portion (5b) without self-locking.

3. A device according to Claim 2,
**characterised in that** the bore (13) of the guide bush (10) is designed with a conical guide face (13b) corresponding to the conical guide portion (5b) of the neck part (5).

4. A device according to Claim 2,
**characterised in that** the cylindrical guide face (13a) of the bore (13) extends over a longitudinal portion of the guide bush (10) housing the conical guide portion (5b) of the neck part (5).

5. A device according to Claim 4,
**characterised in that** the neck part (5) is designed with a second conical guide portion (5c),
**and in that** the bore (13) of the guide bush (10) is designed with a correspondingly conical guide face (13c).

6. A device according to one of Claims 2 to 5,
**characterised in that** the neck part (5) is designed with a cylindrical end portion (5d),
**and in that** the bore (13) is designed with a corresponding second cylindrical guide face (13d).

7. A device according to one of the preceding Claims,
**characterised in that** the stop face (15) of the handle (7) is constructed at slip-on part (14) covering at least a part of the cross section of the bore (13) of the guide bush (10),
**and in that** the neck part (5) comprises at least one guide face (18) placed transversally to its support face (16) and extending in the direction of the centring axis (Z) of the neck part (5), for a counter-face (20) constructed at the slip-on part (14).

8. A device according to Claim 7,
**characterised in that** the support face (16) of the neck part (5) is constructed at a set-back portion of the front face (17),
**in that** the guide face (18) is constructed at a shoulder of the neck part (5) limiting the support face (16),
**and in that** the counter-face (20) is constructed on a corresponding shoulder of the slip-on part (14).

9. A device according to Claim 7 or 8,
**characterised in that** the guide face (18) is constructed on a flank of a groove-like cavity (37) constructed in the support face (16) of the neck part (5),
**and in that** the counter-face (20) is constructed on a guide part which protrudes from the slip-on part (14) and can be inserted into this cavity (37).

10. A device according to one of the preceding Claims,
**characterised in that** the detent pawl is provided with a spring element (34), which under initial stress can abut a stop (35) constructed at the handle (7), and can be braced via this against the locking position.

11. A device according to Claim 10,
**characterised in that** the detent pawl and the spring element (34) are constructed at a single-piece component.

12. A device according to one of the preceding Claims,
**characterised in that** the detent pawl is disposed in the region of a recess (26) constructed in the guide bush (10).

13. A device according to one of the preceding Claims,
**characterised in that** the claw (27) of the detent pawl is designed with a flank (28) turned towards the stop face (15) of the handle (7), which flank is intended to cooperate with a corresponding counter-flank (31) constructed at the recess (30) of the neck part (5) of the rasp (2) and faced towards to the shaft body (4).

14. A device according to Claim 13,
**characterised in that** the flank (28) of the claw (27) and the counter-flank (31) of the recess (30) of the neck part (5) are each constructed in the form of an undercut cylindrical face, which is designed with a radius of curvature (R) which corresponds to the distance of the flank (28) from the swivel axis (A) of the detent pawl.

15. A device according to one of the preceding Claims,
**characterised in that** the claw (27) of the detent pawl is designed with a flank (40), remote from the stop face (15) of the handle (7), in the form of an oblique leading face, which is intended to interact with the end of the neck part (5) when setting down the guide bush (10) and via which the claw (27), when placing the guide bush (10) on the neck part (5), is swivelled increasingly into the disengaged position, which permits an engagement of the claw (27) in the recess (30) when the stop face (15) and the support face (16) meet.

## Revendications

1. Dispositif de préparation d'un os long pour la mise en place d'une tige d'implant à munir d'une articulation sphérique artificielle, notamment d'un corps de fémur d'une prothèse d'articulation de la hanche, avec une râpe (2) correspondant de par sa forme et sa grandeur à la tige d'implant et qui présente une tige de corps (4) pouvant être enfoncée et retirée dans la direction de son axe longitudinal (L) dans l'os (1) et une partie de col (5) dépassant de celui-ci, pouvant être pourvue d'une boule d'essai (6) correspondant à l'articulation sphérique, et une partie de préhension (7) pouvant être placée sur la râpe (2) qui comporte un manchon (10) avec un perçage (13) pour la réception de la partie de col (5) et un dispositif de verrouillage pouvant être relié relâchablement à la partie de col (5), où la partie de col (5) est réalisée avec une surface d'appui (16) au côté frontal pour une surface de butée (15) réalisée à la partie de préhension (7), pouvant être placée, pour l'enfoncement de la râpe (2), sur cette surface d'appui (16) et où le dispositif de verrouillage comporte une partie d'accouplement (11) disposée de manière réglage à la partie de préhension (7), pouvant être mise en prise avec la partie de col (5) et pouvant être éloignée de celle-ci, caractérisé en ce que le manchon (10) est réalisé comme manchon de guidage pouvant être poussé sur la partie de col (5) de la râpe (2) et que la partie d'accouplement (11) est réalisée sous la forme d'un cliquet de blocage articulé de manière pivotante à la partie de préhension (7) qui comporte une griffe (27) déplaçable entre une position de blocage à l'intérieur d'un évidement (30) réalisé à la partie de col (5) de la râpe (2) et une position de relâchement à l'extérieur de cet évidement.

2. Dispositif selon la revendication 1, caractérisé en ce que la partie de col (5) est réalisée avec un tronçon de guidage (5b) prévu pour coopérer avec un perçage de réception conique de la boule d'essai (6), diminuant d'une manière conique relativement à un axe de centrage (Z), en ce qu'il est réalisé en outre à la partie de col (5) un tronçon de guidage cylindrique (5c) pouvant être inséré dans le manchon de guidage (10), en ce que le perçage (13) du manchon de guidage (10) comporte une surface de guidage cylindrique (13a) correspondant au tronçon de guidage cylindrique (5a) et en ce que, lorsque la partie de préhension est placée sur la surface d'appui (16) de la partie de col (5), la partie de col (5) est guidée dans le perçage (13) du manchon de guidage (10) avec un jeu qui assure un centrage du manchon de guidage (10) sur la partie de col (5) et qui permet en même temps un relâchement du tronçon de guidage conique (5b) sans autoblocage.

3. Dispositif selon la revendication 2, caractérisé en ce que le perçage (13) du manchon de guidage (10) est réalisé avec une surface de guidage conique (13b) correspondant au tronçon de guidage conique (5b) de la partie de col (5).

4. Dispositif selon la revendication 2, caractérisé en ce que la surface de guidage cylindrique (13a) du perçage (13) s'étend sur un tronçon longitudinal, recevant le tronçon de guidage conique (5b) de la partie de col (5), du manchon de guidage (10).

5. Dispositif selon la revendication 4, caractérisé en ce que la partie de col (5) est réalisée avec un deuxième tronçon de guidage conique (5c) et en ce que le perçage (13) du manchon de guidage (10) est réalisé avec une surface de guidage conique (13c) correspondante.

6. Dispositif selon l'une des revendications 2 à 5, caractérisé en ce que la partie de col (5) est réalisée avec un tronçon d'extrémité cylindrique (5d) et en ce que le perçage (13) est réalisé avec une deuxième surface de guidage cylindrique correspondante (13d).

7. Dispositif selon l'une des revendications précédentes, caractérisé en ce que la surface de butée (15) de la partie de préhension (7) est réalisée à une partie de pose (14) recouvrant au moins une partie de la section transversale du perçage (13) du manchon de guidage (10), et en ce que la partie de col (5) est réalisée avec au moins une surface de guidage (18) orientée transversalement à sa surface d'appui (16), s'étendant en direction de l'axe de centrage (Z) de la partie de col (5), pour une contre-surface (20) réalisée à la partie de pose (14).

8. Dispositif selon la revendication 7, caractérisé en ce que la surface d'appui (16) de la partie de col (5) est réalisée dans un tronçon décalé vers l'arrière de la surface frontale (17), en ce que la surface de guidage (18) est réalisée à un épaulement, délimitant la surface d'appui (16), de la partie de col (5) et en ce que la contre-surface (20) est réalisée à un épaulement correspondant de la partie de pose (14).

9. Dispositif selon la revendication 7 ou 8, caractérisé en ce que la surface de guidage (18) est réalisée à un flanc d'un creux (37) configuré en rainure réalisé dans la surface d'appui (16) de la partie de col (5), et en ce que la contre-surface (20) est réalisée à une partie de guidage dépassant de la partie de pose (14), insérable dans ce creux (37).

10. Dispositif selon l'une des revendications précédentes, caractérisé en ce que le cliquet d'arrêt est pourvu d'un élément élastique (34) applicable sous précontrainte à une butée (35) réalisée à la partie de préhension (7) et peut être mis en tension par celui-ci dans la position de blocage.

11. Dispositif selon la revendication 10, caractérisé en ce que le cliquet d'arrêt et l'élément élastique (34) sont réalisés à un composant en une pièce.

12. Dispositif selon l'une des revendications précédentes, caractérisé en ce que le cliquet d'arrêt est disposé au voisinage d'un évidement (26) réalisé dans le manchon de guidage (10).

13. Dispositif selon l'une des revendications précédentes, caractérisé en ce que la griffe (27) du cliquet d'arrêt est réalisée avec un flanc (28) orienté vers la surface de butée (15) de la partie de préhension (7), qui est prévu pour la coopération avec un contre-flanc correspondant (31), réalisé à l'évidement (30) de la partie de col (5) de la râpe (2), orienté vers le corps de tige (4).

14. Dispositif selon la revendication 13, caractérisé en ce que le flanc (28) de la griffe (27) et le contre-flanc (31) de l'évidement (30) de la partie de col (5) sont réalisés chacun sous la forme d'une surface cylindrique à contre-dépouille qui est réalisée avec un rayon de courbure (R) qui correspond à l'écart du flanc (28) relativement à l'axe de pivotement (A) du cliquet d'arrêt.

15. Dispositif selon l'une des revendications précédentes, caractérisé en ce que la griffe (27) du cliquet d'arrêt est réalisée avec un flanc (40) détourné de la surface de butée (15) de la partie de préhension (7), sous forme d'une surface de montée oblique qui est prévue pour la coopération avec l'extrémité de la partie de col (5) lors de la mise en place du manchon de guidage (10) et par laquelle la griffe (27), lors de la mise en place du manchon de guidage (10) sur la partie de col (5) peut être pivotée progressivement dans la position de relâchement qui, lors de la rencontre de la surface de butée (15) et de la surface d'appui (16) permet un enclenchement de la griffe (27) dans l'évidement (30).
